# EUROPEAN PATENT APPLICATION

(11) **EP 4 813 443 A1**
(43) Date of publication of application: **30.09.2026**
(21) Application number: 25165646.8
(22) Date of filing: 24.03.2025
(51) Int. Cl.: A61N 1/368, A61B 5/364, A61N 1/362

(54) **IMPLANTABLE MEDICAL DEVICE ENABLING A TEMPORARY REDUCTION OF A PROTECTIVE INTERVAL**

(71) Applicant: BIOTRONIK SE & Co. KG, 12359 Berlin (DE)
(72) Inventor: Schneider, Peter, 12527 Berlin (DE); Fischer, René, 10245 Berlin (DE); Busch, Ulrich, 10318 Berlin (DE); Kucher, Andreas, 16303 Schwedt (DE)
(74) Representative: Biotronik Corporate Services SE

(57) **Abstract**

The present invention relates to an implantable medical device (1) for stimulating a human or animal heart. During operation, the implantable medical device (1) executes a method comprising the following steps: a) detecting at least one right ventricular depolarization (RVs) with a first electrode (5) or delivering at least one right ventricular pacing pulse (RVp) with the first electrode (5); b) determining if a scheduled left ventricular pacing pulse (LVp) can be delivered with a second electrode (9), wherein a delivery of the left ventricular pacing pulse (LVp) is subject to an expiration of a presettable protective interval (LVURI) that is initiated by a preceding left ventricular pacing pulse (LVp) delivered with the second electrode (9), a preceding left ventricular depolarization (LVs) detected with the second electrode (9), or a preceding logic event (LVp(PH)) triggered instead of a preceding left ventricular pacing pulse (LVp) at the same time at which the preceding left ventricular pacing pulse (LVp) was scheduled to be delivered; and c) if the left ventricular pacing pulse (LVp) cannot be delivered since the presettable protective interval (LVURI) is not yet expired , reducing a following presettable protective interval (LVURI) such that it elapses before a respective subsequent left ventricular pacing pulse (LVp) is scheduled.

## Description

The present invention relates to an implantable medical device according to the preamble of claim 1 and to a method for operating an implantable medical device according to the preamble of claim 14.

Implantable medical devices for stimulating a human or animal heart, such as pacemakers, have been known for a long time. They can perform different functions. Different stimulation programs can be carried out by an appropriate pacemaker to restore the treated heart to a normal state.

To give an example, pacemakers can be used for cardiac resynchronization therapy (CRT) in which they apply stimulation pulses to a de-synchronized heart in order to resynchronize the heart again.

In pacemaker modes with programmed biventricular or left ventricular pacing a situation can occur (e. g., by an atrial or a ventricular extrasystole or by a change of timing parameters of the pacing program) that leads to an inhibition of the delivery of a left ventricular pace. If due to the missing pace an occurring left ventricular depolarization (i.e., a left ventricular sense event, LVs) retriggers a protective interval, then the next left ventricular pace delivery can be inhibited again and so on. A so-called lock-in situation is generated. This phenomenon is also described, e.g., by Barold and Kucher (Barold, S. Serge, and Andreas Kucher. "Interruption of cardiac resynchronization therapy by atrial premature complexes." Journal of Electrocardiology 51.2 (2018): 247-251).

Prior art pacemakers employing CRT pursue different strategies to avoid such a lock-in situation. Some of the prior art pacemakers do not enable the retriggering of the protective interval by left ventricular sense events. However, this reduces the medical safety of such CRT devices irrespective of the cardiac rhythm of the patient. If, e.g., the parameter "LV T-wave protection" (that prevents a left ventricular stimulation during the T wave which could lead to ventricular fibrillation) is turned off permanently, there is an increased risk of left ventricular pacing into the vulnerable phase of the left ventricle. This can have severe side effects for the patient.

It is an object of the present invention to provide a device for cardiac resynchronization therapy that enables safe cardiac resynchronization therapy also in case of an irregular cardiac rate and avoids pacing situations that increase the risk of cardiac fibrillation.

This object is achieved with an implantable medical device for stimulating a human or animal heart according to claim 1. Such an implantable medical device comprises a processor, a memory unit, a stimulation unit, and a detection unit. The stimulation unit serves for stimulating a human or animal heart. The detection unit serves for detecting an electric signal of the same heart, i.e., a cardiac electric signal. The detection unit comprises a first electrode for detecting right ventricular electric signals and a second electrode for detecting left ventricular electric signals.

According to an aspect of the present invention, the memory unit comprises a computer-readable program that causes the processor to perform the steps explained in the following when being executed on the processor.

In a first step, it is determined if a scheduled left ventricular pacing pulse can be delivered with the second electrode. This delivery is subject to a condition.

According to the condition, a presettable protective interval needs to be expired. This presettable protective interval has two functions. First, it defines a maximum left ventricular pacing rate. Under this aspect, the presettable protective interval is utilized to prevent sensible patients from experiencing palpitations at elevated resynchronization rates. Second, since the present implantable medical device is capable of left ventricular sensing, the presettable protective interval serves as a protection against competitive left ventricular pacing after detected left ventricular senses. Expressed in other words, the presettable protective interval allows the delivery of a left ventricular pacing pulse only if the left ventricle is in a physiologic state in which a contraction would occur under natural conditions. The presence of the protective interval thus improves the well-being of the patient. The presettable protective interval is initiated by one of three possible events. The first event is a preceding left ventricular pacing pulse delivered with the second electrode. The second event is a preceding left ventricular depolarization detected with the second electrode. The third event is a preceding logic event that is triggered instead of a preceding left ventricular pacing pulse at the same time at which the preceding left ventricular pacing pulse was scheduled to be delivered. Such a logic event can also be denoted as phantom pace or phantom pacing pulse. It replaces a real pacing pulse in case that the conditions for delivering the real pacing pulse are not met (e.g., because of a non-terminated presettable protective interval). In this context, "preceding" refers to an event that directly or indirectly precedes the left ventricular pacing pulse scheduled to be delivered (but being subject to the conditions to be met).

If the left ventricular pacing pulse scheduled to be delivered cannot be delivered since the presettable protective interval is not yet expired, a following presettable protective interval to be started after occurrence of one of the precedingly explained three events (in particular after occurrence of a left ventricular depolarization) is shortened so as to elapse before a respective subsequent left ventricular pacing pulse is scheduled. This will enable the delivery of a following left ventricular pacing pulse aiming in a cardiac resynchronization even in case that a right ventricular extrasystole or a left ventricular extrasystole has occurred that resulted in a cardiac de-synchronization and a premature start of the presettable protective interval.

By shortening a following presettable protective interval, a cardiac resynchronization can be restored after the synchronizing event. This increases the general CRT success. Furthermore, it helps to reduce the number of the synchronization episodes after the detection of CRT interrupt. At the same time, the permanent LV T-wave protection remains basically fully active. Moreover, a time period in which a patient may experience palpitations due to a temporarily reduced protective interval is minimized. During higher cardiac rates, the CRT pacing is even faster resynchronized than during lower cardiac rates.

Prior art CRT devices currently record intracardiac electrogram (IEGM) episodes of the type "end of CRT pacing". Patients having an irregular cardiac rate generate a plurality of such IEGM episodes (partly even everyday) that are entered into a log file or that are sent to a remote service center. These IEGM episodes are even generated if a statistic evaluation shows almost 100 % CRT pacing per day. Expressed in other words, these IEGM episodes result from an undesired temporal interruption of CRT due to the lacking possibility of a fast resynchronization of the heartbeat. It would generally be desirable to reduce the number of such IEGM episodes to a minimum to clearly identify critical IEGM episodes or to not store or present rather uncritical IEGM episodes representing a (short) interruption of CRT. By enabling fast resynchronization after a de-synchronizing event due to a shortening of the presettable protective interval, the number of such IEGM episodes can be significantly reduced, wherein only IEGM episodes that could not be resolved by the performed shortening of the presettable protective interval would be recorded at all. This reduces the amount of physiologically irrelevant IEGM episodes and allows an easy identification of relevant IEGM episodes that require separate or additional therapy.

In an embodiment, the computer-readable program causes the processor to detect one right ventricular depolarization with the first electrode or to deliver at least one right ventricular pacing pulse with the first electrode, and to carry out steps a) and b) upon detecting the at least one right ventricular depolarization or upon delivering the at least one right ventricular pacing pulse (RVp). Adding such an additional step of sensing a right ventricular depolarization or of delivering a right ventricular pacing pulse upon which steps a) and b) are carried out is particularly advantageous, if the implantable medical device is operated in a BiV-RV operation mode where a leading pacing pulse is delivered in the right ventricle, since a resynchronisation effect may be improved or accelerated.

In an embodiment, the computer-readable program causes the processor to shorten only the next presettable protective interval or 2 to 10, in particular 3, 4 or 5 following consecutive presettable protective intervals. By limiting the shortening to only a single presettable protective interval, the time period in which a patient may experience palpitations due to a temporarily reduced protective interval is considerably low, in particular in comparison to permanently shortening the parameter "LV T-wave protection" according to prior art solutions. Otherwise by extending the shortening of the presettable protective interval to 2 to 6 following consecutive cardiac cycles, a persistent risk of an LVURI lock-in situation may be prevented without analyzing the timing situation anew thereby saving computational and time resources.

In an embodiment, the computer-readable program causes the processor to repeat the step of determining if a left ventricular pacing pulse can be delivered and the step of reducing a following presettable protective interval in 1 to 6, in particular 2 to 5, in particular 3 to 4 subsequent cardiac cycles. This facilitates re-establishing resynchronization of the heart, e.g., during a phase in which the ventricular rate would otherwise inhibit a scheduled left ventricular pacing pulse due to a premature start of the presettable protective interval and/or an overlap of two or more presettable protective intervals that result in an extended period of time during which left ventricular pacing pulses are inhibited. However, one should bear in mind that a repetition of the individual method steps might increase the risk of experiencing palpitations and thus deteriorate the well-being of the patient due to an applied left ventricular pacing pulse. Therefore, the repetition should not exceed the above-mentioned number.

In an embodiment, the computer-readable program causes the processor to determine if a re-establishing of cardiac resynchronization has been successful. In case that no cardiac resynchronization could yet be obtained, the step of determining if a left ventricular pacing pulse can be delivered (upon fulfilment of the above-mentioned condition), and the step of reducing a following presettable protective interval are repeated after a predeterminable first time period or upon a reduction of the current heart rate. The predeterminable first time period represents a pause in the applied intervention for attempting the re-establishing of cardiac synchronization.

In an embodiment, the first time period lies in a range of from 30 seconds to 5 minutes, in particular from 45 seconds to 4 minutes, in particular from 1 minute to 3.5 minutes, in particular from 1.5 minutes to 3 minutes, in particular from 2 minutes to 2.5 minutes. The alternative trigger for repeating the individual method steps, i.e., a reduction of the current heart rate, is, in an embodiment, a reduction of the current heart rate by at least 10 bpm, in particular at least 15 bpm, in particular at least 20 bpm, in particular at least 25 bpm, in particular at least 30 bpm. In an embodiment, the reduction of the current heart rate is a reduction lying in a range of from 10 bpm to 55 bpm, in particular from 15 bpm to 50 bpm, in particular from 20 bpm to 45 bpm, in particular from 25 bpm to 40 bpm, in particular from 30 bpm to 35 bpm.

In an embodiment, the computer-readable program causes the processor to perform the step of determining if a left ventricular pacing pulse can be delivered (upon fulfilment of the above-mentioned condition), and the step of reducing the next or one or more following presettable protective intervals only 1 to 6 times, in particular only 2 to 5 times, in particular only 3 to 4 times per predeterminable time window ranging from 6 minutes to 1 hour, in particular from 10 minutes to 55 minutes, in particular from 15 minutes to 50 minutes, in particular from 20 minutes to 45 minutes, in particular from 25 minutes to 40 minutes, in particular from 30 minutes to 35 minutes. By applying such a limitation of attempts for achieving resynchronization of the heart over a specific time window, the risk that the applied intervention interferes with the health status of the patient is significantly reduced. Thus, a limitation of attempts over the specified time window represents a good compromise between attempting to achieve a resynchronization of the heart and reducing the risk of experiencing palpitations by fast left ventricular pacing.

In an embodiment, the computer-readable program causes the processor to perform the step of determining if a left ventricular pacing pulse can be delivered (upon fulfilment of the above-mentioned condition), and the step of reducing the next or one or more following presettable protective intervals only if at least 2, in particular at least 3, in particular at least 4, in particular at least 5, in particular at least 6, in particular at least 7, in particular at least 8, in particular at least 9, in particular at least 10, in particular exactly 2, 3, 4, 5, 6, 7, 8, or 10, consecutive scheduled left ventricular pacing pulses cannot be delivered for reason that the respective presettable protective intervals have not been expired prior to the scheduled pulse delivery. By increasing the number of consecutive scheduled left ventricular pacing pulses, the delivery of which could not take place, the sensitivity of the applied algorithm is reduced so that the attempt of applying a cardiac resynchronization is only performed in case of a persisting cardiac de-synchronization. This will allow additional time for the heart to return to its physiologic synchronized periodic beating pattern without external intervention (i.e., without applying the left ventricular pacing pulse). This increases the physiologic compatibility of the implantable medical device and its functionality.

In an embodiment, the computer-readable program causes the processor to disable the step of reducing the next or one or more following presettable protective intervals for a predeterminable second time period if cardiac resynchronization has not been successful during a third time period. In this context, the third time period occurs earlier than the second time period. Disabling the step of reducing the next or one or more following presettable protective intervals is a safety measure to avoid the consecutive attempt of application resynchronization therapy to the patient if the patient is not susceptible to such therapy. In such a case, it is more convenient to disable the functionality and to enable it again only after a follow-up examination of the patient by a physician or after the expiration of a specific time period during which the physiologic state of the patient's heart can have changed so that the heart becomes (again) more susceptible to the resynchronization therapy to be applied.

In an embodiment, an unsuccessful cardiac resynchronization is assumed if N attempts for establishing cardiac resynchronization by reducing the presettable protective interval during the third time period did not result in cardiac resynchronization. In this context, N is a number lying in a range of from 20 to 1500, in particular from 30 to 1400, in particular from 40 to 1300, in particular from 50 to 1200, in particular from 60 to 1100, in particular from 75 to 1000, in particular from 90 to 900, in particular from 100 to 800, in particular from 200 to 700, in particular from 300 to 600, in particular from 400 to 500.

In an embodiment, the third time period lies in a range of from 6 hours to 24 hours, in particular from 7 hours to 23 hours, in particular from 8 hours to 22 hours, in particular from 9 hours to 21 hours, in particular from 10 hours to 20 hours, in particular from 11 hours to 19 hours, in particular from 12 hours to 18 hours, in particular from 13 hours to 17 hours, in particular from 14 hours to 16 hours, in particular from 15 hours to 15.5 hours.

In an embodiment, the second time period lies in a range of from 1 to 7 days, in particular from 2 to 6 days, in particular from 3 to 5 days, in particular from 4 to 4.5 days, or lasts until the next scheduled follow-up examination of the patient.

In an embodiment, the computer-readable program causes the processor to perform steps a) to b) for a maximum number of 2 to 10, in particular 3 or 5 cardiac cycles and disable step b) for 40 to 60, in particular 48 cardiac cycles upon reaching the maximum number. In another embodiment, the computer-readable program causes the processor to perform steps a) to b) for 10 to 15 cardiac cycles, in particular 12 cardiac cycles within a total number of 40 to 60 cardiac cycles, in particular 48 cardiac cycles and disable step b) for 140 to 180 cardiac cycles, in particular for 160 cardiac cycles.

In an embodiment, the presettable protective interval has a duration lying in a range of from 275 ms to 700 ms, in particular from 300 ms to 675 ms, in particular from 325 ms to 675 ms, in particular from 350 ms to 650 ms, in particular from 375 ms to 625 ms, in particular from 400 ms to 600 ms, in particular from 425 ms to 575 ms, in particular from 450 ms to 550 ms, in particular from 475 ms to 525 ms or from 275 ms to 400 ms. A duration of the presettable protective interval of 300 ms corresponds to a cardiac rate of 200 bpm. I.e., as long as the cardiac rate is not higher than 200 bpm, than a reduction of the presettable protective interval to 300 ms will allow the delivery of a left ventricular pacing pulse. A duration of the presettable protective interval of 700 ms corresponds to a cardiac rate of approximately 86 bpm. The intermediate variations of the presettable protective intervals correspond to intermediate cardiac rates. The cardiac rate in beats per minute can be calculated as 1/(duration in milliseconds)*60,000. The reduced presettable protective interval has a duration at the lower end of the beforementioned range, e.g., in a range of from 300 ms to 400 ms, in particular from 325 ms to 375 ms. The regular (non-reduced) presettable protective interval has a duration at the upper end of the beforementioned interval, e.g., in a range from 500 ms to 700 ms, in particular from 550 ms to 675 ms, in particular from 600 ms to 650 ms.

In an embodiment, the presettable protective interval may be reduced to a length which is smaller than the difference of the current cardiac interval and a time between a logic event triggered instead of the scheduled left ventricular pacing pulse and a left ventricular depolarization initiating the following presettable protective interval. This length reduction of the presettable protective interval enables that the following presettable protective interval is expired at the time the respective subsequent left ventricular pacing pulse is scheduled.

In an embodiment, the implantable medical device is operable in a first mode in which a left ventricular pacing pulse is scheduled for delivery after an expiration of a pacing interval or in a second mode in which a left ventricular pacing pulse is scheduled for delivery after an expiration of a presettable interventricular interval which lies in a range of from 0 ms to 100 ms, or has a length of 5 ms or 20 ms. The interventricular interval represents the regular delay of contraction of one (left or right) ventricle in relation to a contraction of the other ventricle. The interventricular interval is typically rather short since both the right ventricle and the left ventricle depolarize quite simultaneously.

In an embodiment, the implantable medical device comprises a data communication unit that serves for transferring data to a remote monitoring system in a wireless manner. All standard data transmission protocols or specifications are appropriate for such a wireless data communication. Examples of standard data transmission protocols or specifications are the Medical Device Radiocommunications Service (MICS), the Bluetooth Low Energy (BLE) protocol, the Zigbee specification, the long range wide area network (LoRaWAN) protocol, the wireless personal area network (WPAN) specification, the low-power wide-area network (LPWAN) specification, the wireless local area network (WLAN) specification, the Global System for Mobile Communications (GSM) specification, the Long-Term Evolution (LTE) standard, and the fifth-generation technology standard for broadband cellular networks (5G). In an aspect, the present invention relates to a method for operating an implantable medical device for stimulating a human or animal heart, in particular an implantable medical device according to the preceding explanations. In this context, the method comprises the steps explained in the following.

In a first step, it is determined if a scheduled left ventricular pacing pulse can be delivered with a second electrode. This delivery is subject to a condition.

According to the condition, a presettable protective interval needs to be expired. This presettable protective interval serves for preventing a left ventricular pacing into a vulnerable phase of the left ventricle. The presettable protective interval is initiated by one of three possible events. The first event is a preceding left ventricular pacing pulse delivered with the second electrode. The second event is a preceding left ventricular depolarization detected with the second electrode. The third event is a preceding logic event that is triggered instead of a preceding left ventricular pacing pulse at the same time at which the preceding left ventricular pacing pulse was scheduled to be delivered. Such a logic event can also be denoted as phantom pace or phantom pacing pulse. It replaces a real pacing pulse in case that the conditions for delivering the real pacing pulse are not met (e.g., because of a non-terminated presettable protective interval). In this context, "preceding" refers to an event that directly or indirectly precedes the left ventricular pacing pulse scheduled to be delivered (but being subject to the conditions to be met).

In a second step, a following presettable protective interval to be started upon occurrence of one of the precedingly explained three events (in particular upon occurrence of a left ventricular depolarization) is shortened so as to elapse before a respective subsequent left ventricular pacing pulse is scheduled, if the left ventricular pacing pulse scheduled to be delivered cannot be delivered since the presettable protective interval is not yet expired. This will enable the delivery of the following left ventricular pacing pulse aiming in a cardiac resynchronization even in case that a right ventricular extrasystole or a left ventricular extrasystole has occurred that resulted in a cardiac de-synchronization and a premature start of the following presettable protective interval.

In an aspect, the present invention relates to a method for providing a cardiac resynchronization therapy to a patient in need thereof with an implantable medical device for stimulating a human or animal heart, in particular an implantable medical device according to the preceding explanations. In this context, the method comprises the steps explained in the following.

In a first step, it is determined if a scheduled left ventricular pacing pulse can be delivered with a second electrode. This delivery is subject to a condition.

According to the condition, a presettable protective interval needs to be expired. This presettable protective interval serves for preventing a left ventricular pacing into a vulnerable phase of the left ventricle. The presettable protective interval is initiated by one of three possible events. The first event is a preceding left ventricular pacing pulse delivered with the second electrode. The second event is a preceding left ventricular depolarization detected with the second electrode. The third event is a preceding logic event that is triggered instead of a preceding left ventricular pacing pulse at the same time at which the preceding left ventricular pacing pulse was scheduled to be delivered. Such a logic event can also be denoted as phantom pace or phantom pacing pulse. It replaces a real pacing pulse in case that the conditions for delivering the real pacing pulse are not met (e.g., because of a non-terminated presettable protective interval). In this context, "preceding" refers to an event that directly or indirectly precedes the left ventricular pacing pulse scheduled to be delivered (but being subject to the conditions to be met).

In a second step, a following presettable protective interval to be started upon occurrence of one of the precedingly explained three events (in particular upon occurrence of a left ventricular depolarization) is shortened so as to elapse before a respective subsequent left ventricular pacing pulse (LVp) is scheduled, if the left ventricular pacing pulse scheduled to be delivered cannot be delivered since the presettable protective interval is not yet expired. This will enable the delivery of the following left ventricular pacing pulse aiming in a cardiac resynchronization even in case that a right ventricular extrasystole or a left ventricular extrasystole has occurred that resulted in a cardiac de-synchronization and a premature start of the following presettable protective interval.

**In** a third step, the respective subsequent left ventricular pacing pulse is delivered to the patient after termination of the reduced following presettable protective interval.

All embodiments of the implantable medical device can be combined in any desired way and can be transferred either individually or in any arbitrary combination to the described method. Likewise, all embodiments of the described method can be combined in any desired way and can be transferred either individually or in any arbitrary combination to the implantable medical device.

Further details of aspects of the present invention will be explained in the following making reference to exemplary embodiments and accompanying Figures. **In** the Figures:
- Figure 1A: schematically shows a system comprising an implantable medical device;
- Figure 1B: schematically shows different components of the implantable medical device of Figure 1A;
- Figure 2A: shows a first schematic illustration of sensed and stimulated cardiac events as part of a therapy using a prior art CRT device together with selected timing parameters of the CRT device;
- Figure 2B: shows a second schematic illustration of sensed and stimulated cardiac events as part of a therapy using an exemplary embodiment of a CRT device according to the present disclosure together with selected timing parameters of the CRT device;
- Figure 3A: shows a third schematic illustration of sensed and stimulated cardiac events as part of a therapy using a prior art CRT device together with selected timing parameters of the CRT device; and
- Figure 3B: shows a fourth schematic illustration of sensed and stimulated cardiac events as part of a therapy using an exemplary embodiment of a CRT device according to present disclosure together with selected timing parameters of the CRT device.

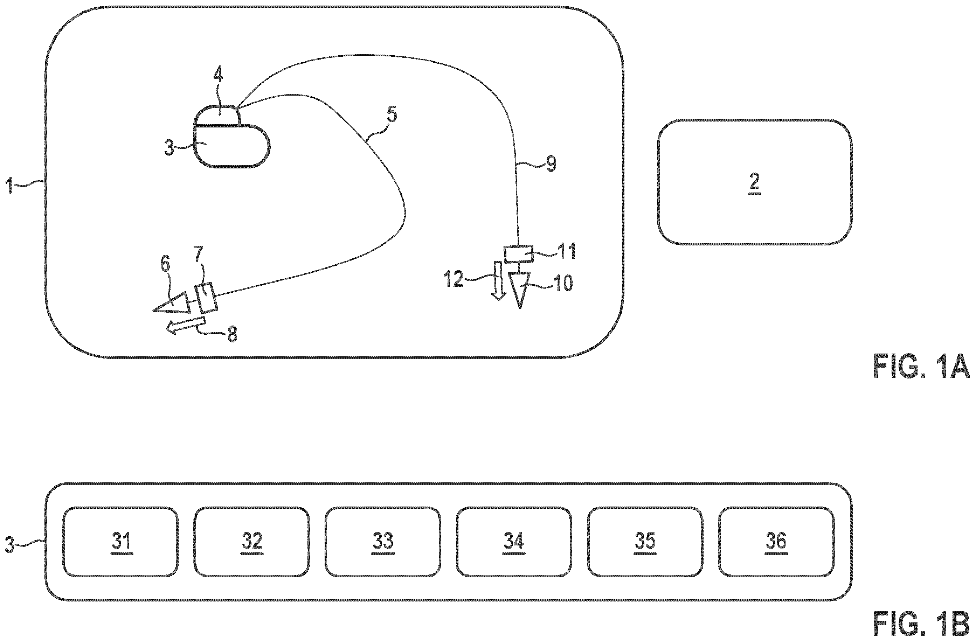

Figure 1A shows a system comprising a device for cardiac resynchronization therapy (CRT) 1 as example of an implantable medical device for stimulating the human or animal heart. The system further comprises a home monitoring system 2 serving as remote system. It is possible for the CRT device 1 to establish a wireless data communication with the home monitoring system 2.

The CRT device 1 comprises a housing 3 with a header 4 and a first electrode 5 connected to the header 4. The first electrode 5 comprises a first tip electrode pole 6 and a first ring electrode pole 7 that is proximally arranged from the first tip electrode pole 6. A first sensing vector 8 is defined from the first ring electrode pole 7 to the first tip electrode pole 6. Electric cardiac signals sensed between the first tip electrode pole 6 and first the ring electrode pole 7, i.e., along the first sensing vector 8, are directly recorded within a heart chamber, typically the right ventricle. Thus, the first electrode 5 is designed and arranged to sense right ventricular electric signals.

The CRT device 1 additionally comprises a second electrode 9 that is also connected to the header 4. The second electrode 9 comprises a second tip electrode pole 10 and a second ring electrode pole 11 that is proximally arranged from the second tip electrode pole 10. A second sensing vector 12 is defined from the second ring electrode pole 11 to the second tip electrode pole 10. Electric cardiac signals sensed between the second tip electrode pole 10 and the second ring electrode pole 11, i.e., along the second sensing vector 12, are also directly recorded within a heart chamber, typically the left ventricle. Thus, the second electrode 9 is designed and arranged to sense left ventricular electric signals.

Figure 1B schematically illustrates individual components of the CRT device 1 that are comprised within the housing 3. In this and in all following Figures, similar elements will be denoted with the same numeral reference. The housing 3 houses a detection unit 31 (also referred to as sensing unit) that typically comprises an analog-to-digital converter, a bandpass filter, and an offset compensation. The detection unit 31 is operatively connected with a processor 32 that has access to a memory unit 33. The memory unit 33 serves for storing instructions for the processor 32 as well as data detected by the detection unit 31. The housing 3 further comprises an evaluation unit 34 that can also be part of the processor 32 and that serves for extracting features from the detected cardiac electric signal. The housing 3 further comprises a stimulation unit 35 that serves for stimulating the heart from which the detection unit 31 detects electric signals. The first electrode 5 along with its first tip electrode pole 6 and first ring electrode pole 7 as well as the second electrode 9 along with its second tip electrode 10 and second ring electrode 11 (confer Figure 1A) form part of the detection unit 31 and of the stimulation unit 35. Additionally, the housing 3 comprises a data communication unit 36 that serves for data transfer to the home monitoring system 2 (confer Figure 1A).

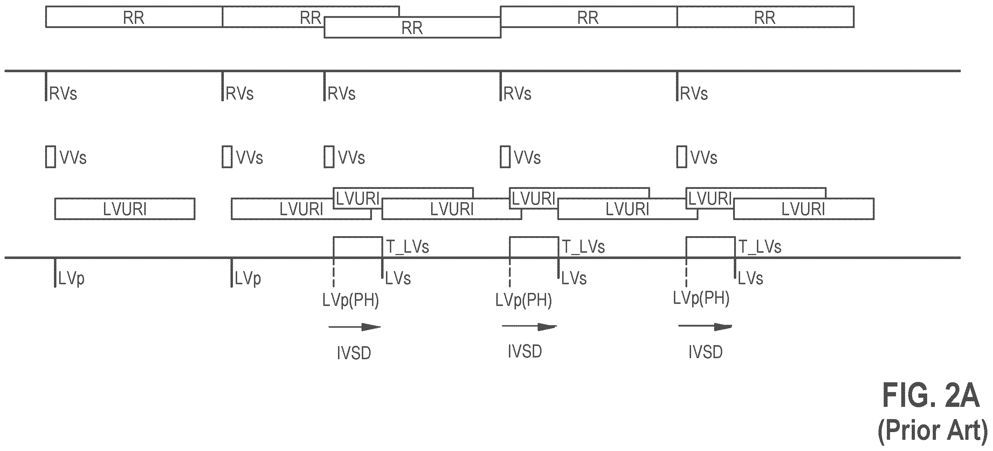

Figure 2A shows a first schematic illustration of sensed and stimulated cardiac events as part of a therapy using a prior art CRT device together with selected timing parameters of the CRT device. The CRT device is programmed to be operated as VVIR BiV-LV type device (i.e., a device with ventricular stimulation, ventricular sensing, inhibited operating mode, and a rate-adaptive frequency adaptation with biventricular stimulation, wherein the first paced ventricle is the left ventricle).

After a right ventricular depolarization RVs has been detected, a small interventricular interval VVs passes until a left ventricular resynchronization pacing pulse LVp is applied by the CRT device. In other words, the left ventricular resynchronization pulse LVp is scheduled to be delivered upon expiration of the interventricular interval VVs. Since this left ventricular pacing pulse is applied interventricular (IVC), the pulse is also denoted as LVp.

With the delivery of this left ventricular resynchronization pacing pulse LVp, the left ventricular upper resynchronization interval LVURI (an embodiment of the presettable protective interval) is started. The length of the left ventricular upper resynchronization interval LVURI is adjusted to the cardiac rate of the patient's heart to be treated with the CRT device. Typically, the left ventricular upper resynchronization interval LVURI ends prior to the next sensed right ventricular depolarization RVs. Thus, after another interventricular interval VVs, a novel left ventricular resynchronization pacing pulse LVp is delivered by the CRT device. Consequently, a novel left ventricular upper resynchronization interval LVURI starts. The time between two subsequent right ventricular depolarizations is also denoted as RR interval and indicated in Figure 2A with the letters "RR".

In case of the premature right ventricular depolarization RVs (either due to a right ventricular extrasystole or due to an increased cardiac rate), the subsequent interventricular interval VVs ends within a persisting left ventricular upper resynchronization interval LVURI. During this left ventricular upper resynchronization interval LVURI, the delivery of a further left ventricular pacing pulse is inhibited. Consequently, only a logical event, namely a left ventricular resynchronization phantom pacing pulse LVp(PH) is emitted. In addition, a left ventricular timing window T_LVs is initiated that lasts until the detection of an intrinsic left ventricular depolarization LVs. The time between the right ventricular depolarization RVs and the left ventricular depolarization LVs is also referred to as interventricular sense delay IVSD.

Both the left ventricular resynchronization phantom pacing pulse LVp(PH) and the sensed left ventricular depolarization LVs initiate further left ventricular upper resynchronization intervals LVURI. Consequently, different left ventricular upper resynchronization intervals LVURI overlap and inhibit the further delivery of a left ventricular resynchronizing pacing pulse LVp. A so-called LVURI lock-in situation has occurred that persistently prevents left ventricular resynchronization pacing. It will not be possible for the CRT device to terminate the LVURI lock-in situation. Thus, the CRT device cannot perform its cardiac resynchronization functionality any longer.

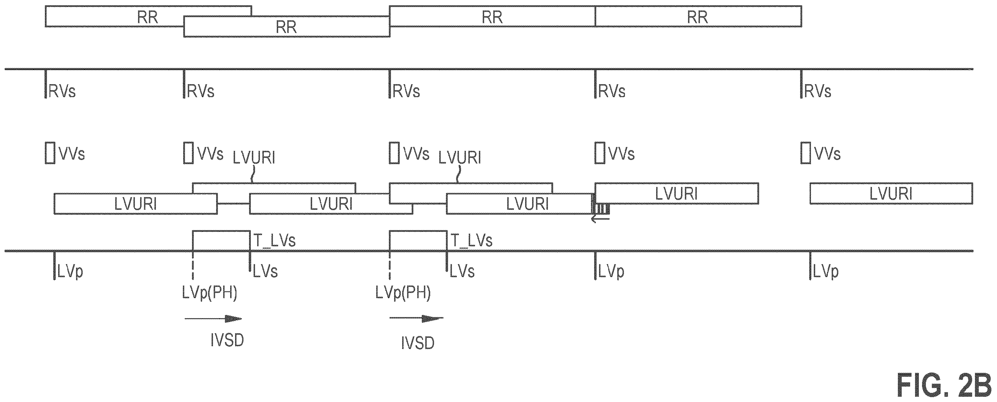

Figure 2B shows a similar schematic illustration, however, not for a prior art device, but for an embodiment of an implantable medical device according to the present disclosure. This implantable medical device is a CRT device that is programmed to be operated as VVIR BiV-LV type device (like the CRT device in Figure 2A). Here, a premature right ventricular depolarization RVs also initiates another left ventricular upper resynchronization interval LVURI at a time point at which the preceding left ventricular upper resynchronization interval LVURI has not yet terminated. Consequently, the delivery of a scheduled left ventricular resynchronization pacing pulse LVp is inhibited. Instead, a left ventricular resynchronization phantom pacing pulse LVp(PH) is emitted. Subsequently, an intrinsic left ventricular depolarization LVs can be detected which starts a novel left ventricular upper resynchronization interval LVURI. Thus, the situation is almost the same as in case of the prior art device.

However, upon detecting that a scheduled left ventricular resynchronization pacing pulse LVp could not have been delivered due to an ongoing left ventricular upper resynchronization interval LVURI, the CRT device shortens the following left ventricular upper resynchronization interval LVURI, as illustrated by the arrow pointing to the left side below the fourth sensed right ventricular depolarization RVs. This shortening or reduction of the left ventricular upper resynchronization interval LVURI results in a termination of this interval upon or prior to the detection of the fourth right ventricular depolarization RVs. Consequently, the next scheduled left ventricular resynchronization pacing pulse LVp can be delivered after expiration of the interventricular interval VVs so that a resynchronization of the heart can take place. The delivered left ventricular resynchronizing pacing pulse LVp then starts a novel left ventricular upper resynchronization interval LVURI that has the original length (i.e., it is longer than the preceding, reduced left ventricular upper resynchronization interval LVURI). Afterwards, the heartbeat will take place in a regular and synchronized manner.

The reduction of the left ventricular upper resynchronization interval LVURI is automatically performed by the processor by applying a preset value which is reduced compared to the regular value for the left ventricular upper resynchronization interval LVURI. This preset value is selected once by a user e.g. by a responsible physician during a follow-up examination and then stored as the reduced LVURI. The processor repeatedly automatically applies this reduced LVURI when performing the step of reducing a following presettable protective interval (LVURI) such that it elapses before a respective subsequent left ventricular pacing pulse is scheduled until another preset value is selected by the physician and stored. In an advantageous embodiment, the reduction of the left ventricular upper resynchronization interval LVURI is performed in dependence on the actual cardiac rate of the patient's heart. In particular, the reduced left ventricular upper resynchronization interval LVURI may advantageously be reduced to a length which is smaller than the difference of the current cardiac interval and the interventricular sense delay IVSD. This aims to the shortened left ventricular upper resynchronization interval LVURI being terminated upon or before the next right ventricular depolarization RVs is detected.

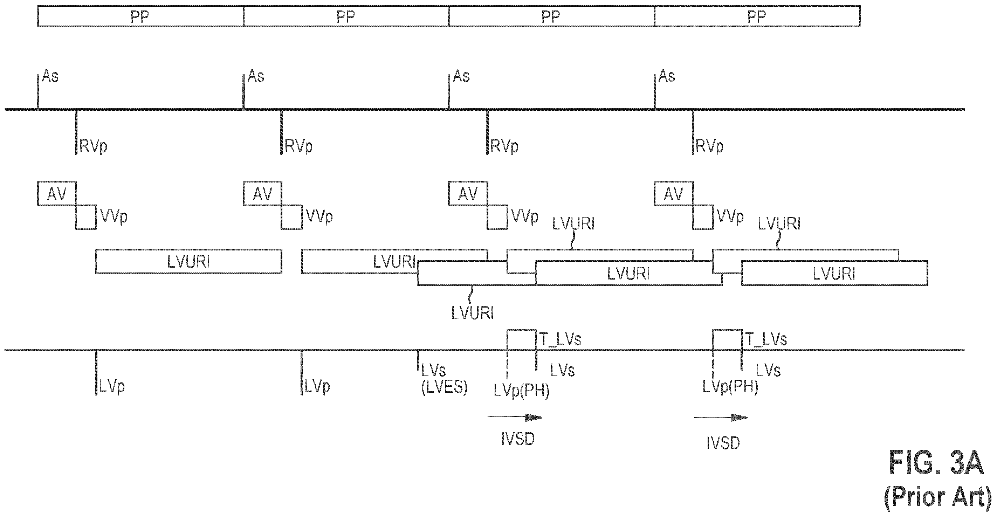

Figure 3A shows another schematic illustration of sensed and stimulated cardiac events as part of a therapy of a prior art CRT device together with selected timing parameters of the CRT device. In this comparative example, the CRT device is programmed as DDD BiV-RV type device (i.e., it employs a dual stimulation, a dual sensing, and a dual (inhibiting and triggered) operating mode; in addition, it performs a biventricular stimulation, wherein the right ventricle is the first paced ventricle).

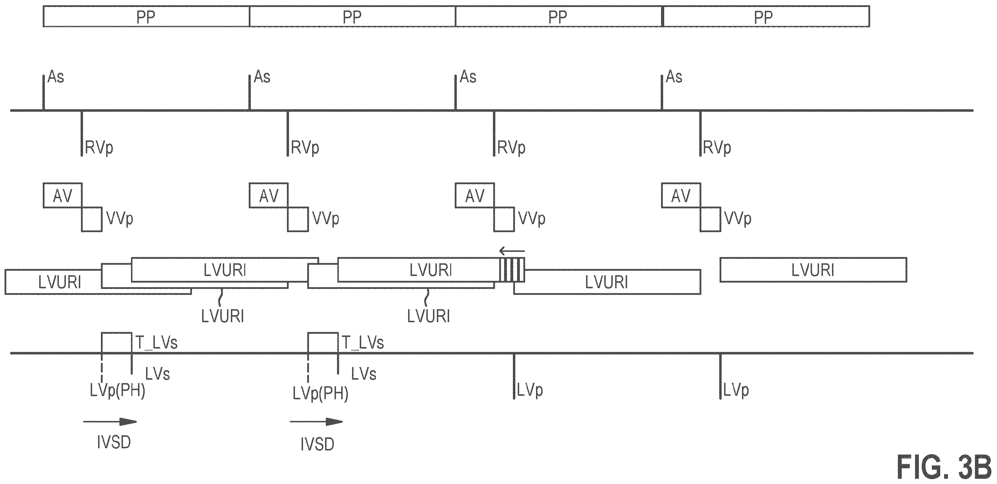

In this example, an atrial sense As is detected. After expiration of the atrioventricular interval AV (also referred to as atrioventricular delay), a right ventricular pacing pulse RVp is delivered. After a pacing-initiated interventricular interval VVp has expired, a left ventricular resynchronizing pacing pulse LVp is delivered to the left ventricle. This results in the start of a left ventricular upper resynchronization interval LVURI. After detecting another atrial event As, the same sequence of events takes place. The interval between two atrial sensings (i.e., between two atrial events, also referred to as P wave), i.e., the PP interval, is indicated in Figures 3A and 3B by the letters "PP".

Then, however, a left ventricular extrasystole LVES leads to a left ventricular depolarization LVs. Consequently, a novel left ventricular upper resynchronization interval LVURI is started, wherein the previous left ventricular upper resynchronization interval LVURI has not yet been terminated. The next scheduled left ventricular resynchronization pacing pulse cannot be delivered since the previously started left ventricular upper resynchronization interval LVURI is still active. As a consequence, only a left ventricular resynchronizing phantom pacing pulse LVp(PH) is emitted. This leads to the start of a novel left ventricular upper resynchronization interval LVURI. Shortly afterwards, namely after elapsing of an interventricular sense delay IVSD, a left ventricular depolarization LVs is sensed in response to the previously applied right ventricular pacing pulse RVp. This left ventricular depolarization LVs starts another left ventricular upper resynchronization interval LVURI.

Consequently, three different left ventricular upper resynchronization interval LVURI overlap. One of these left ventricular upper resynchronization intervals LVURI also persists at the time at which the next left ventricular resynchronizing pacing pulse LVp is scheduled so that once again only a left ventricular resynchronizing phantom pacing pulse LVp(PH) is emitted. Thus, the device has turned into an LVURI lock-in situation from which it cannot return by itself.

Figure 3B shows a similar situation like that of Figure 3A, however, using a CRT device being an embodiment of the present disclosure. This CRT device is also operated as DDD BiV-RV type device. The schematic illustration of Figure 3B starts in a cardiac cycle in which an LVURI lock-in situation has already occurred due to an ongoing left ventricular left ventricular upper resynchronization interval LVURI at the time at which a left ventricular resynchronizing pacing pulse LVp was scheduled. As a result, only a left ventricular resynchronizing phantom pacing pulse LVp(PH) is emitted that starts another left ventricular upper resynchronization interval LVURI. A subsequent left ventricular depolarization LVs starts another left ventricular upper resynchronization interval LVURI that persists at a time point at which the succeeding left ventricular pacing pulse was scheduled but is now inhibited. The emitted left ventricular resynchronizing phantom pacing pulse LVp(PH) starts a novel left ventricular upper resynchronization interval LVURI. Furthermore, the next intrinsic left ventricular depolarization LVs starts also a left ventricular upper resynchronization interval LVURI.

The CRT device has now detected that two left ventricular resynchronizing pacing pulses could not have been delivered as scheduled. Rather, two consecutive left ventricular resynchronizing phantom pacing pulses LVp(PH) have been emitted instead. This triggers a reduction of the following left ventricular upper resynchronization interval LVURI that would persist without reduction to a time point at which the next left ventricular pacing pulse is intended to be delivered. This is illustrated by the arrow pointing to the left side below the third delivered right ventricular pacing pulse RVp. Due to the reduction of this left ventricular upper resynchronization interval LVURI in dependence on the detected cardiac rate and the interventricular sense delay IVSD, this left ventricular upper resynchronization interval LVURI is terminated before the next left ventricular resynchronizing pacing pulse LVp is scheduled to be delivered. Consequently, this pacing pulse LVp can be delivered as scheduled. This leads to a resynchronization of the right ventricular depolarization and the left ventricular depolarization, i.e., to a cardiac resynchronization. The left ventricular upper resynchronization interval LVURI that is initiated by the delivered left ventricular resynchronizing pacing pulse LVp regularly terminates prior to the next scheduled left ventricular pacing pulse so that the CRT device has left the LVURI lock-in situation and is able to provide the required cardiac resynchronization therapy, i.e., its normal functionality.

As illustrated by the preceding examples, the presently claimed and described implantable medical device is able to automatically terminate an LVURI lock-in situation that occurred due to a temporarily reduced cardiac rate and thus to return to its normal functionality without external intervention.

## Claims

1. Implantable medical device (1) for stimulating a human or animal heart, comprising a processor (32), a memory unit (33), a stimulation unit (35) configured to stimulate a human or animal heart, and a detection unit (31) configured to detect an electric signal of the same heart, wherein the detection unit (31) comprises a first electrode (5) for detecting right ventricular electric signals and a second electrode (9) for detecting left ventricular electric signals,
**characterized**
**in that** the memory unit (33) comprises a computer-readable program that causes the processor (32) to perform the following steps when being executed on the processor (32):
a) determining if a scheduled left ventricular pacing pulse (LVp) can be delivered with the second electrode (9), wherein a delivery of the left ventricular pacing pulse (LVp) is subject to an expiration of a presettable protective interval (LVURI) that is initiated by a preceding left ventricular pacing pulse (LVp) delivered with the second electrode (9), a preceding left ventricular depolarization (LVs) detected with the second electrode (9), or a preceding logic event (LVp(PH)) triggered instead of a preceding left ventricular pacing pulse (LVp) at the same time at which the preceding left ventricular pacing pulse (LVp) was scheduled to be delivered;
b) if the left ventricular pacing pulse (LVp) cannot be delivered, since the presettable protective interval (LVURI) is not yet expired, reducing a following presettable protective interval (LVURI) such that it elapses before a respective subsequent left ventricular pacing pulse (LVp) is scheduled.

2. Implantable medical device according to claim 1, **characterized in that** the computer-readable program causes the processor (32) to detect one right ventricular depolarization (RVs) with the first electrode (5) or to deliver at least one right ventricular pacing pulse (RVp) with the first electrode (5); and to carry out steps a) and b) upon detecting the at least one right ventricular depolarization (RVs) or upon delivering the at least one right ventricular pacing pulse (RVp).

3. Implantable medical device according to any one of the preceding claims, **characterized in that** the computer-readable program causes the processor (32) to reduce only the next presettable protective interval (LVURI) or 2 to 10, in particular 3, following consecutive presettable protective intervals (LVURI).

4. Implantable medical device according to any of the preceding claims, **characterized in that** the computer-readable program causes the processor (32) to repeat steps a) to c) 1 to 6 times.

5. Implantable medical device according to any of the preceding claims, **characterized in that** the computer-readable program causes the processor (32) to determine if a reestablishing of cardiac resynchronization has been successful and, if it has not been successful, to repeat steps a) to b) after a predeterminable first time period lying in a range of from 30s to 2 min or upon a reduction of the current heart rate.

6. Implantable medical device according to any of the preceding claims, **characterized in that** the computer-readable program causes the processor (32) to perform steps a) to b) only 1 to 6 times per predeterminable time window ranging from 5 minutes to 1 hour.

7. Implantable medical device according to any of the preceding claims, **characterized in that** the computer-readable program causes the processor (32) to perform steps a) to b), only if at least 2 to 10, in particular 3, consecutive scheduled left ventricular pacing pulses (LVp) cannot be delivered for reason that the respective presettable protective intervals (LVURI) have not been expired prior to the scheduled pulse delivery.

8. Implantable medical device according to any of the preceding claims, **characterized in that** the computer-readable program causes the processor (32) to disable step b) for a predeterminable second time period, if a cardiac resynchronization has not been successful during a third time period.

9. Implantable medical device according to claim 8, **characterized in that** an unsuccessful cardiac resynchronization is assumed, if N attempts for establishing cardiac resynchronization by reducing the presettable protective interval (LVURI) during the third time period did not result in cardiac resynchronization, wherein N is a number lying in a range of from 20 to 1500.

10. Implantable medical device according to claim 8 or 9, **characterized in that** the third time period lies in a range of from 6 hours to 24 hours, and/or the second time period lies in a range of from 1 day to 7 days or extends to a scheduled follow-up examination.

11. Implantable medical device according to any of the preceding claims, **characterized** in thatthe computer-readable program causes the processor (32) to perform steps a) to b) for a maximum number of 2 to 6, in particular 3 cardiac cycles and disable step b) for 40 to 60, in particular 48 cardiac cycles upon reaching the maximum number and/or to perform steps a) to b) for 10 to 15 cardiac cycles, in particular 12 cardiac cycles within a total number of 40 to 60 cardiac cycles, in particular 48 cardiac cycles and disable step b) for 140 to 180 cardiac cycles, in particular for 160 cardiac cycles.

12. Implantable medical device according to any of the preceding claims, **characterized in that** the presettable protective interval (LVURI) has a duration lying in a range of from 275 ms to 400 ms, or has a length of 325 ms.

13. Implantable medical device according to any one of the preceding claims, **characterized in that** the implantable medical device is operable in a first mode in which a left ventricular pacing pulse (LVp) is scheduled for delivery after an expiration of a pacing interval or in a second mode in which a left ventricular pacing pulse (LVp) is scheduled for delivery after an expiration of a presettable interventricular interval (VVs, VVp) which lies in a range of from 0 ms to 100 ms, or has a length of 20 ms.

14. Method for operating an implantable medical device (1) for stimulating a human or animal heart, in particular an implantable medical device (1) according to any of the preceding claims, **characterized by** the following steps:
a) determining if a scheduled left ventricular pacing pulse (LVp) can be delivered with a second electrode (9), wherein a delivery of the left ventricular pacing pulse (LVp) is subject to an expiration of a presettable protective interval (LVURI) that is initiated by a preceding left ventricular pacing pulse (LVp) delivered with the second electrode (9), a preceding left ventricular depolarization (LVs) detected with the second electrode (9), or a preceding logic event (LVp(PH)) triggered instead of a preceding left ventricular pacing pulse (LVp) at the same time at which the preceding left ventricular pacing pulse (LVp) was scheduled to be delivered;
b) if the left ventricular pacing pulse (LVp) cannot be delivered since the presettable protective interval (LVURI) is not yet expired, reducing a following presettable protective interval (LVURI) such that it elapses before a respective subsequent left ventricular pacing pulse (LVp) is scheduled.

15. Method for providing a cardiac resynchronization therapy to a patient in need thereof with an implantable medical device (1) for stimulating a human or animal heart, in particular an implantable medical device (1) according to any of claims 1 to 13, the method comprising the following steps:
a) determining if a scheduled left ventricular pacing pulse (LVp) can be delivered with a second electrode (9), wherein a delivery of the left ventricular pacing pulse (LVp) is subject to an expiration of a presettable protective interval (LVURI) that is initiated by a preceding left ventricular pacing pulse (LVp) delivered with the second electrode (9), a preceding left ventricular depolarization (LVs) detected with the second electrode (9), or a preceding logic event (LVp(PH)) triggered instead of a preceding left ventricular pacing pulse (LVp) at the same time at which the preceding left ventricular pacing pulse (LVp) was scheduled to be delivered;
b) if the left ventricular pacing pulse (LVp) cannot be delivered, since the presettable protective interval (LVURI) is not yet expired, reducing a following presettable presettable protective interval (LVURI) such that it elapses before a respective subsequent left ventricular pacing pulse (LVp) is scheduled to obtain a reduced protective interval (LVURI);
c) delivering the following left ventricular pacing pulse (LVp) to the patient after termination of the reduced presettable protective interval (LVURI).
